(19) **Europäisches Patentamt**
European Patent Office
Office européen des brevets

(11) **EP 0 732 924 B1**

(12) **EUROPÄISCHE PATENTSCHRIFT**

(45) Veröffentlichungstag und Bekanntmachung des Hinweises auf die Patenterteilung:
**10.06.2009 Patentblatt 2009/24**

(21) Anmeldenummer: **95903793.8**

(22) Anmeldetag: **06.12.1994**

(51) Int Cl.:
*A61P 39/00* (2006.01)          *A61K 9/70* (2006.01)
*A61K 45/06* (2006.01)          *A61K 31/407* (2006.01)
*A61K 31/46* (2006.01)

(86) Internationale Anmeldenummer:
**PCT/EP1994/004048**

(87) Internationale Veröffentlichungsnummer:
**WO 1995/015755 (15.06.1995 Gazette 1995/25)**

(54) **PARASYMPATHIKOMIMETIKUM UND PARASYMPATHIKOLYTIKUM ENTHALTENDES TTS**

TTS CONTAINING A PARASYMPATHOMIMETIC AND A PARASYMPATHOLYTIC AGENT

TTS COMPRENANT UN AGENT PARASYMPATHOMIMETIQUE ET UN AGENT PARASYMPATHOLYTIQUE

(84) Benannte Vertragsstaaten:
**AT BE CH DE DK ES FR GB GR IE IT LI LU NL PT SE**
Benannte Erstreckungsstaaten:
**SI**

(30) Priorität: **10.12.1993 DE 4342174**

(43) Veröffentlichungstag der Anmeldung:
**25.09.1996 Patentblatt 1996/39**

(73) Patentinhaber: **LTS LOHMANN Therapie-Systeme AG**
**56626 Andernach (DE)**

(72) Erfinder:
• **HILLE, Thomas**
  **D-56564 Neuwied (DE)**
• **MÜLLER, Walter**
  **D-56564 Neuwied (DE)**
• **ASMUSSEN, Bodo**
  **D-22949 Ammersbek (DE)**

(74) Vertreter: **Flaccus, Rolf-Dieter**
**Flaccus · Müller-Wolff**
**Patentanwälte**
**Bussardweg 10**
**50389 Wesseling (DE)**

(56) Entgegenhaltungen:
WO-A-93/03767          US-A- 4 031 894
US-A- 4 788 063          US-A- 4 952 586

• JOURNAL CANADIEN DE PHYSIOLOGIE ET PHARMACOLOGIE, Bd.64, Nr.7, 1986 Seiten 1047 - 9 SHILOFF ET AL 'Effects of subchronic pyridostigmine pretreatment on the toxicity of soman'
• GOV. REP. ANNOUNCE INDEX, Bd.89, Nr.15, 1989 SOLANA ET AL 'Comparing the Efficacy of Physostigmine Pretreatment in Combination With Scopolamine Versus Artane Against Coman Challenge'
• PROG. NEURO-PSYCHOPHARMACOL. & BIOL. PSYCHIAT., Bd.14, 1990 Seiten 83 - 90 CASSONE ET AL 'Effects of Combinations of Arecoline and Atropine on Mouse Motor Activity'
• LIFE SCIENCES, Bd.40, 1987 Seiten 577 - 83 HARRIS ET AL 'Oxime-induced decarbamylation and atropine/oxime therapy of guinea pigs intoxitaed with pyridostigmine'
• S.T.P. PHARMA, Bd.1, Nr.1, 1985 Seiten 25 - 36 SÉGOT-CHICQ ET AL 'Les dispositifs à liBöration contrôlée pour la délivrance des principes actifs médicamenteux; I. Intérêt et applications'
• LEVY ET AL: 'Proceedings of the Forth Intl. Symposium on Protection Against Chemical Warefare Agents, 8.-12.06.1992: Human Studies With Transdermal Physostigmine', 1992, NATIONAL RESEARCH DEFENSE ESTABLISHMENT, SWEDEN * Seite 277 - Seite 284 *
• GOSDEN ET AL: '4. Medizinische C-Schutz-Tagung des Bundesministeriums der Verteidigung (BMVG)": Effects of transdermal physostigmine and hyoscine on physiology and performance', * Seite 151 - Seite 156 *

EP 0 732 924 B1

**Beschreibung**

Die Erfindung betrifft ein transdermales therapeutisches System sowie ein Verfahren zur kombinierten transdermalen Anwendung von Physostigmin und Scopolamin für die Prophylaxe bzw. Vorbehandlung einer Vergiftung durch hochtoxische phosphororganische Cholinesterasehemmer, insbesondere Soman.

Insbesondere soll die vorliegende Erfindung pharmazeutische formulierungen zur Verfügung stellen, die zur prophylaktischen Behandlung von Vergiftungen durch phosphororganische Cholinesterasehemmer geeignete Wirkstoffe ohne schädliche Nebenwirkungen in kontrollierter Weise freisetzen.

Zur Gruppe der phosphororganischen Cholinenterasehemmer gehören bestimmte Ester von Phosphorsäurederivaten wie z.B. Nitrostigmin (= Diethyl-(4-nitrophenyl)-thiophosphat), besser unter den Bezeichnungen Parathion oder E 605 bekannt, aber auch Tabun sowie die Phosphonsäurederivate Sarin, Soman und VX.

Cholinesterasehemmenda Phosphorsäureester werden unter anderem in der Landwirtschaft als Insektizide eingesetzt, da sie auch für Menschen toxisch sind, besteht eine grundsätzliche Gefaher für Leib und Leben von Mitarbeitern im agrikulturellen Bereich, dies umso mehr, als diese organischen Phosphorsäureester auch über die Haut aufgenommen werden können. Gegenüber den Insektiziden zeichnen sich die zu den sogenannten Nervenkampfstoffen zählenden Verbindungen Tabun, Sarin, Soman und VX durch eine besonders hohe Toxizität aus.

Alle diese Verbindungen sind mehr oder weniger starke Hemmstoffe der Acetylcholinesterase, eines Enzyms, das physiologisch betrachtet die Wirkung der an bestimmten Nervenenden freigesetzten Überträgersubstanz Acetylcholin beendet. Die Mehrzahl der durch Cholinesterasehemmer verursachten Vergiftungssymptome ist durch eine Überschwemmung mit körpereigenem Acetylcholin bedingt.

Die medikamentöse Grundbehandlung einer solchen Vergiftung besteht in der Verabreichung des-Parasympatikolytikums Atropin, wodurch die überschießenden muskarinischen Acetylcholin-Wirkungen (z.B. Sekretionssteigerung in den Atemwegen, Bronchospasmus, Hemmung der zentralnervösen Atemantriebs) blockiert werden. Zur Normalisierung der überschießenden nikotinischen Acetylcholin-Wirkungen (z.B. Hemmung der Erregungsübertragungen an den Synapsen motorischer Nerven zur Atem- und übrigen Skelettmuskulatur bis zur vollständigen peripheren Muskellähmung) steht kein geeigneter Antagonist zur Verfügung. Die peripher ausgelöste Muskellähmung läßt sich nur mit Oximen wie z.B. Pralidoxim (PAM) oder Obidoxim (Toxogonin®) aufheben, deren Wirkungsmechanismus in einer Reaktivierung der gehemmten Acetylcholinesterase besteht.

Diese Postexpositionstherapie reicht jedoch nicht aus, das überlegen nach einer Vergiftung mit der zweifachen $LD_{50}$ Soman ($LD_{50}$ = Dosis, die für 50% der Exponierten tödlich ist) zu sichern. Die Überlebenswahrscheinlichkeit nach einer Soman-Vergiftung steigt nur dann, wenn bereits vor der Giftexposition mit einem Carbamat, z.B. Pyridostigmin oder Physostigmin, vorbehandelt worden ist und außerdem die herkömmliche Antidot-Therapie mit Atropin und einem Oxim unverzüglich eingeleitet wird, sobald die ersten Vergiftungesymptome auftreten. Bezüglich des zur Vorbehandlung eingesetzten Carbamate ist zu fordern, daß es bei möglichst hoher, langanhaltender Schutzwirkung keine ins Gewicht fallende unerwünschten Wirkungen aufweist, insbesondere die Reaktionsfähigkeit nicht beeinträchtigt.

Einige phosphororganische Cholinesterasehemmer zeichnen sich dadurch aus, daß sie nach Anlagerung an die Acotylcholinesterase Alkylreste abspalten, wodurch sich die Bindung stabilisiert ("Alterung"). Der gealterte Esterase-Hemmstoff-Komplex läßt sich durch Oxime nicht reaktivieren. Bei Vergiftungen mit dem Nervenkampfstoff Soman dritt die Alterung bereits nach 2 bis 5 Minuten ein. Die Therapie mit Atropin und Oximen kann jedoch durch Vorbehandlung mit indirekten Parasympathikomimetika, z.B. Carbaminsäureestern wie Pyridostigmin und Physostigmin, wesentlich verbessert werden.

Carbaminsäureester hemmen die Acetylcholinesterase in ähnlicher weise wie Phosphorsäure. Die Bindung ist allerdings kurzfristiger und vollständig reversibel. Für die Schutzwirkung von Carbamaten dürfte ausschlaggebend sein, daß die in geeigneter Dosierung einen Teil der Äcetylcholinesterase hemmen und damit den Zugriff der stärker anhaltend hemmenden Phosphor- und Phosphonsäureester entziehen, wenn die Vorbehandlung rechtzeitig erfolgte.

Auch die Behandlung einer Vergiftung durch phosphorische Insektizide erfordert in jedem Fall ärztliche Betreuung, die rasch eingeleitet werden muß. Weil rasche ärztliche Hilfe im Falle von Erntearbeitern nicht immer gegeben ist, besteht Bedarf an Medikamenten, die prophylaktisch einer Intoxikation entgegenwirken. Der Einsatz von Carbaminsäureestern für diesen Zweck ist beschrieben (Leadbeater, L. Chem. in Brit. 24, 683, 1988). Gleiches gilt für die Wirksamkeit von Carbaminsäureestern zur Vorbehandlung einer Soman-Vergiftung im Tierexperiment (Fleischer, J.H., Harris, L.W. Biochem. Pharmacol. 14, 641, 1965; Berry, W.K., Davies, D.R. Biochem, Pharmacol. 19, 927 1970). Prophylaktisch anzuwendende Arzneimittel dürften in wirksamer Dosierung Reaktionsvermögen und Leistungsfähigkeit nicht beeinträchtigen. Die therapeutische Breite der Carbaminsäureester ist allerdings gering. Mit Physostigmin läßt sich zwar eine höhere Schutzwirkung erreichen als mit Pyridostigmin, die Nebenwirkungen sind jedoch höher.

Unerwünschte parasympathikomimetische. Wirkungen der Carbamate lassen sich grundsätzlich durch Kombinationen mit einem Parasympathikolytikum (z.B. Atropin, Scopolamin) zurückdrängen.

DE-OS 41 15 558 beschreibt ein prophylaktisches Antidot, bestehend aus einer Kombination aus Pyridostigsain oder Physostigmin und N-methyl-4-piperidyl-1-phenylcyclopentancarboxylat-Hydrochlorid oder Arpenal, Sycotrol, Carmiphen oder Benaktyzin und zusätzlich zwingend einen Tranquilizer, nämlich Diazepam oder Clonazepam. Die unerwünschten Wirkungen von Physostigmin oder Pyridostigmin lassen sich daher nicht allein durch die aufgezählten Parasympäthikolytika unterdrücken, weshalb zusätzlich Tranquilizer gegeben werden, deren Nebenwirkungsprofit ebenfalls-problematisch ist.

Es ist daher erforderlich, die prophylaktische Gabe von Carbaminsäureestern oder anderen indirekten Parasympathikomimetika in einer Dosierung zu ermöglichten, die einen ausreichenden Schutz gegen phosphororganische Cholinesterasehemmer ohne unerwünschte Begleiterscheinungen bewirkt.

Levy et al. beschreiben in Proc. 4th Int. Symp. Protection Against Chemical Warfare Agents, (1992) 277-284 unter dem Titel "Human Studies with Transdermal Physostigmine" Studien zur transdermalen Verabreichung von Physostigmin und Scopolamin an Schweine zur Prophylaxe gegen Vergiftung durch Soman. Den Schweinen wird dazu gleichzeitig zu einem "Physostigmin-Pad" der Wirkstoff Scopolamin mittels des kommerziell erhältlichen Produkts Scopoderm® transdermal appliziert.

Auch in dem Artikel "A Long-Acting Transdermal System for the Treatment of Organophosphate Poisoning" von Levy et al. in Proc. 3rd Int. Symp. Protection Against Chemical Warfare Agents, (1989) 151-156 wird von der Verwendung des kommerziell erhältlichen Scopoderm-TTS in Verbindung mit einem Physostigmin-Präparat berichtet.

Der Erfindung liegt die Aufgabe zugrunde, eine spezielle pharmazeutische Formulierung von Wirkstoffen zur transdermalen Anwendung als Hautpflaster zur möglichst nebenwirkungsfreien Prophylaxe bzw. Vorbehandlung einer Vergiftung durch hochtoxische phosphororganische Cholinesterasehemmer anzugeben, mit folgender Zielsetzung:

-   kontinuierliche gleichmäßige Freisetzung der Wirkstoffe über 72 Stunden hinweg,

-   die Schutzwirkung der Wirkstoffe soll höher sein als die Schutzwirkung von Atropin und reaktivierendem Oxim,

-   unerwünschte Wirkungen, z.B. Leistungsbeeinträchtigungen sollen in der gewählten Dosierung nicht eintreten.

Diese Aufgabe wird erfindungsgemäß gelost durch ein transdermales therapeutisches System mit einer Wirkstoffkombination aus mindestens einem Parasympathikomimetikum und mindestens einem Parasympathikolytikum. Diese Lösung ist umso erstaunlicher, als erfindungsgemäß gezeigt werden kann, daß das Parasympathikolytikum nicht nur zur Schutzwirkung beiträgt, sondern die unerwünschten Wirkungen des Parasyanpathikomimetikums sicher unterdrückt.

Arzneiformen wie transdermale therapeutische Systeme, die Wirkstoffe kontrolliert über längere Zeiträume freisetzen, sind im stand der Technik bekannt.

Auch Systeme, in der die Reservoirschicht so hohe zieehklebrigkeit aufweist, daß sie gleichzeitig die Haftklebeschicht darstellt, sind möglich. Das deutsche Patent DE 38 43 239 beschreibt ein solches System.

Erfindungemäß wird ein Pflastersystem so konstruiert, daß es zwei getrennte Reservoire für das Parasympathikomimetikum und das Parasympathikolytikumbeinhaltet, was ein "zwei in Eins TTS" darstellt. Wenn die Wirkstoffe durch die Haut absorbiert werden, erhält der zur Behandelnde auf diese Weise einen kontrollierten und vorbestimmbaren Zufluß der Wirkstoffe.

Im folgenden wird die Erfindung durch ein Beispiel näher erläutert:

Beispiel:

I. Wirkstofffreies Laminat
Man mischt

-   887,0 kg saure Polyacrylatlösung (50%ig)
-   10,1 kg basisches Methacrylat
-   50,4 kg Triacetin
-   0,508 kg Aluminiumacetylacetonat und
-   37,8 kg Ethanol,

und beschichtet mit dieser Lösung eine durch Silikonisierung wieder ablösbar gemachte Polyesterfolie. Nach Abdampfen der Lösemittel beträgt das Kleberauftragsgewicht 120 g/cm$^2$. Das Laminat wird mit in Längs- und Querrichtung elastischem Trägergewebe aus Polyester abgedeckt (wirkstofffreies Laminat)
II. Scopolamiahaltiges Laminat
Man mischt

- 918,75 kg saure Polyacrylatlösung (50%ig)
- 84,38 kg 1-Dodecanol
- 3,68 kg Acetylaceton
- 3,38 kg Acetylacetonat und
- 18,0 kg Scopolaminbase

und beschichtet mit dieser Lösung eine durch Silikonisierung wieder ablösbar gemachte Polyesterfolie. Nach Verdampfen der Lösemittel beträgt das Kleberauftragsgewicht 150 g/cm$^2$.

Das Laminat wird mit einer 23 $\mu$m dicken Polyesterfolie abgedeckt.

III. Physostigminhaltiges Laminat

Man mischt

- 542,2 kg saure Polyacrylatlösung (50%ig)
- 125,8 kg 1-Dodecanol
- 83,3 kg Physostigmin
- 83,3 kg basisches Methacrylat
- 16,6 kg Aluminiumacetylacetonat und
- 166,6 kg Ethanol

und beschichtet mit dieser Lösung eine durch Silikonisierung wieder ablösbar gemachte PE-Folie. Nach Verdampfen der Lösemittel beträgt das Kleberauftragsgewicht 240 g/cm$^2$. Folgende Schmalrollen werden geschnitten:

| | |
|---|---|
| PE-Folie mit Silikonisierung: | 87 mm breit (I) |
| Wirkstofffreies Laminat: | 87 mm breit (II) |
| Scopolaminhaltiges Laminat: | 15 mm breit (III) |
| Physostigatfnhaltiges Laminat: | 50 mm breit (IV) |

Man entfernt die wiederablösbaren silikonisierten PE-Folien von den scopolamin- und Physostigminhaltigen Laminaten (II und III) und transferiert 15 x 50 mm$^2$ bzw. 50 x 50 mm$^2$ große Rechtecke mit der klebenden Seite mittig und kantenparallel auf die PE-Folie (I).

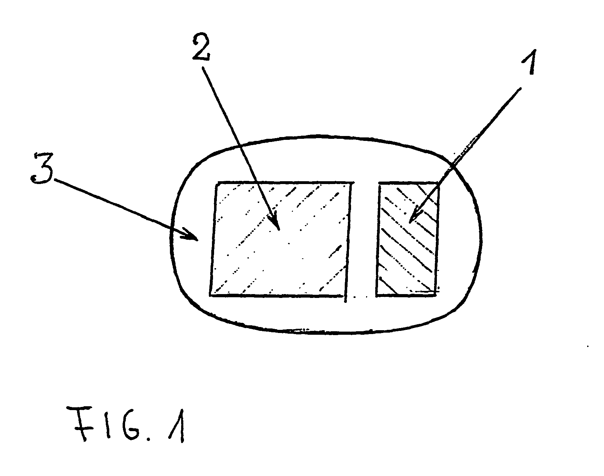

Nun wird vom wirkstofffreien Laminat (I) die wiederablösbare PE-Folie entfernt und Laminat (II) wird mit der klebenden Seite kantengerade über die mit den Rechtecken versehene Bahn (I) kaschiert. Mit einem ovalen Stanzwerkzeug werden die Systeme vereinzelt. In Figur 1 sind die Systeme nach Entfernen der Schutzschicht in Aufsicht dargestellt.

Es bedeuten:

1. Reservoirteil mit Scopolamin
2. Reservoirteil mit Physostigmin
3. Wirkstofffreier Kleberand

In Tabelle 1 und 2 sind die kontrollierten Freisetzungen der Wirkstoffe sowohl in physiologischer Kochsalzlösung als auch durch exzidierte Nagetierhaut dargestellt.

Tabelle 1:

| Kumulierte Freisetzung nach | 2h | 4h | 8h | 24h |
|---|---|---|---|---|
| Scopolamin [mg/cm$^2$] | 0,1 | 0,14 | 0,20 | 0,33 |
| Physostigmin [mg/cm$^2$] | 0,5 | 0,69 | 1,02 | 1,71 |

Tabelle 1: In vitro-Liberation von Scopolamin und Physostigmin
Freisetzungsapparatur: rotierender Zylinder nach US PXXII
Freisetzungsmedium: physiologische Kochsalz lösung
Gehaltsbestimmung mittels HPLC

Tabelle 2:

| Kumulierte Freisetzung nach | 8h | 24h | 48h | 72h |
|---|---|---|---|---|
| Scopolamin [$\mu$g/2,54 cm$^2$] | 5,6 | 67,6 | 200 | - |

(fortgesetzt)

| Kumulierte Freisetzung nach | 8h | 24h | 48h | 72h |
|---|---|---|---|---|
| Physoatigmin [$\mu$g/2,54 cm$^2$] | 95 | 850 | 2160 | 3430 |

Tabelle 2: Penetrationsrate von Scopolamin und Physostigmin
Freisetzungsapparatur Franz-Zelle (Hautmodell Meerschweinhaut)
Freisetzuapsmedium: physiologische Kochsalzlösung
Gehaltsbestimmung mittels HPLC

Die Ergebnisse aus Tabelle 1 belegen die Funktionsfähigkeit des transdermalen therapeutischen Systems gemäß der Erfindung über zwei bzw. drei volle Tage.

Tierexperimentelle Wirksamkeitsprüfung (Referenzversuch)

Gegenüber einer Somanvergiftung an Meerschweinchen wurde die Schutzwirkung von Pyridostigmin und Physostigmin allein sowie in Kombination mit Scopolamin untersucht. Jeweils 6 bis 10 Tiere erhielten 24 h vor der Soman-Belastung ein Pyridostigmin- (3 cm$^2$/kg) oder Physostigmin-Hautpflaster (1,5 cm$^2$/kg). Nach 24 stündiger Anwendung des Physostigmin-Hautpflasters wurden Plasmakonzentrationen von 0,9 $\pm$ 0,3 ng/ml (Mittelwert $\pm$ SEM; n = 4) gemessen. Die Cholinesteraseaktivität im Gesamtblut war bei Anwendung des größeren Pyridostigmin-Hautpflasters um 38 $\pm$ 4% gehemmt, bei Anwendung des kleineren Physostigmin-Hautpflasters um 48 $\pm$ 10%. Zur Prüfung der zusätzlichen Schutzwirkung von Scopolamin wurde entweder ein handelsübliches transdermales therapeutisches System (Scopoderm® TTS) angewendet oder den Tieren wurden osmotische Minipumpen (Alzet®) mit einer Freisetzungsrate von 9 bis 10 ng Scopolaminhydrobromid pro kg Körpergewicht und Stunde subkutan implantiert. Die nach Anwendung des Pyridostigmin- bzw. Physostigmin-Hautpflasters und Soman-Belastung mit 1,5 LD$_{50}$ intramuskulär erzielten Ergebnisse zeigt Tabelle 1.

Die Physostigmin-Vorbehandlung ist nicht nur bei einer Soman- sondern auch bei einer Sarin-Vergiftung wirksam: Nach transdermaler Physostigmin-Scopoderm®-TTS-Vorbehandlung und Belastung mit 1,5 LD$_{50}$ Sarin überlebten 9 von 10 Meerschweinchen ohne zusätzliche Postexpositionstherapie.

In einer zusätzlichen Versuchsreihe an Meerschweinchen wurde die Wirksamkeit der Physostigmin-Vorbehandlung mit und ohne Scopolamin gegenüber Soman bei zusätzlicher Postexpositionstherapie mit Atropinsulfat sowie Obidoximchlorid anhand des Wirksamkeiteindex (protective ratio = Quotient aus LD$_{50}$ mit Behandlung und LD$_{50}$ ohne Behandlung) ermittelt. (Tabelle 2)

Tabelle 1:

| Schutzwirkung verschiedener Vorbehandlungen bei Meerschweinchen gegenüber einer Belastung mit 1,5 LD$_{50}$ Soman i.m. ohne zusätzliche Postexpositionstherapie. | |
|---|---|
| Vorbehandlung | Letalität (24 h) |
| keine | 10/10 |
| Pyridostigmin transdermal (3cm$^2$/kg) | 6/6 |
| Pyridostigmin transdermal (1,5 cm$^2$/kg) + Alzet®-Scopolamin 10 ng kg$^{-1}$h$^{-1}$ | 5/6 |
| Pyridostigmin transdermal (1,5 cm$^2$/kg) | 6/20 |
| Pyridostigmin transdermal (1,5 cm$^2$/kg) + Alzet®-Scopolamia 9 ng kg$^{-1}$h$^{-1}$ | 0/10 |
| Physostigmin transdermal (1,5 cm$^2$/kg) + Scopoderm®-TTS | 1/10 |

Tabelle 2:

| Wirksamkeit der Physostigmin- bzw. kombinierten Physontigmin-Scopolamin-Vorbehandlung bei Meerschweinchen gegenüber Soman-Belastung und zusätzlicher Postexpositionstherapie mit Atropinsulfat und Obidoximchlorid (jeweils 10 mg/kg Körpergewicht i.m. 1 min nach Soman). | |
|---|---|
| Vorbehandlung | Wirksamkeitsindex *) (Vertrauensgrenzen) |
| Physostigmin transdermal (1,5 cm$^2$/kg) | 3,45 |

(fortgesetzt)

| Wirksamkeit der Physostigmin- bzw. kombinierten Physontigmin-Scopolamin-Vorbehandlung bei Meerschweinchen gegenüber Soman-Belastung und zusätzlicher Postexpositionstherapie mit Atropinsulfat und Obidoximchlorid (jeweils 10 mg/kg Körpergewicht i.m. 1 min nach Soman). | |
| --- | --- |
| Vorbehandlung | Wirksamkeitsindex *) (Vertrauensgrenzen) |
| Pyridostigmin transdermal (1,5 cm$^2$/kg) + Alzet®-Scopolamin 4,5 ng kg$^{-1}$h$^{-1}$ | (3,00; 3,95) 3,70 (3, 65; 4,50) |

$$*) \quad \textbf{Wirksamkeitsindex} = \frac{\textbf{LD}_{50} \textbf{ mit Behandlung}}{\textbf{LD}_{50} \textbf{ ohne Behandlung}}$$

Für die kombinierte Vorbehandlung mit transdermalem Physostigmin und Scopoderm®-TTS ohne Postexpositionstherapie ergaben sich in Versuchsreihen mit zwei unterschiedlichen Physostigmin-Formulierungen Wirksamkeitindices von 2,11 (1,71; 2,60) bzw. 2,27 (1,86; 2,79).

Die Pharmakokinetik von transdermal verabreichtem Physostigmin und Scopolamin wurde an Schweinen untersucht. Innerhalb von 5 bis 6 h stieg die Plasmakonzentration auf ein Plateau an, das über 72 h aufrecht erhalten blieb. Für Wirksamkeitsprüfungen an Schweinen gegenüber intravenöser Soman-Belastung wurden Physostigmin-Hautpflaster (0,5 cm$^2$/kg) verwendet, die nach 48 h zu Plasmakonzentrationen von 1,1 $\pm$ 0,1 ng/ml (16 $\pm$ 3% Hemmung der Cholinesteraseaktivität im Gesamtblut) führten. Mit Scopoderm®-TTS wurden Scopolamin-Konzentrationen im Plasma von 0,18 $\pm$ 0,06 ng/ml (n = 9) nach 24 h erreicht. Gegenüber einer Belastung mit 2,5 LD$_{50}$ Soman ergaben sich ohne zusätzliche Postexpositionstherapie folgende Befunde (Tabelle 3):

Tabelle 3:

| Schutzwirkung der Physostigmin- bzw. Physostigsnin-Scopolamin-Vorbehandlung bei Schweinen gegenüber einer Belastung mit 2,5 LD$_{50}$ Soman i. v. ohne zusätzliche Postexpositionstherapie. | | |
| --- | --- | --- |
| Vorbehandlung | Letalität | mittlere Erholungszeit *) (min) |
| Scopoderm®-TTS | 4/4 | — |
| Physostigmin transdermal (0,5 cm$^2$/kg) | 1/4 | 146 |
| Physostigmin transdermal (0,5 cm$^2$/kg) + Scopoderm®-TTS | 2/5 | 29 |
| *) Erholungszeit = Zeit, bis die überlebenden Tiere stehen und laufen können. | | |

Wurden Schweine nach transdermaler Physostigmin-Scopolamin-Vorbehandlung nicht mit 2, 5 LD$_{50}$, sondern mit 4 LD$_{50}$ Soman i.v. belastet und wurde 20 s später eine Postexpositionstherapie durchgeführt (0,5 mg Atropinsulfat sowie 3 mg obidoximchlorid/kg Körpergewicht i.m.), überlebten 3 von 5 Tieren, wobei die überlebender höhere Physostigmin- und Scopolamin-Konzentrationen aufwiesen als die Nicht-tiberlebenden. Enthielt die Postexpositionstherapie zusätzlich Loprazolam (0,2 mg/kg i.m.), überlebten alle 5 Tiere, die Erholung war allerdings bei 2 Tieren unzureichend, was die Nachteile der Benzodiazepingabe exemplarisch darstellt.

Klinische Verträcrlichkeitsuntersuchungen (Referenzversuch)

Die Verträglichkeit von Pbysostigmin-Hautpflastern wurde an 11 freiwilligen Probanden (Alter 29 $\pm$ 2 Jahre) unter Doppelblindbedingungen gegenüber Placebo und zusätzlicher Anwendung von Scopoderm®-TTS geprüft. Bei Phyostigmin-Konzentrationen im Plasma von 0,3 $\pm$ 0,1 ng/ml nach 48 h und Scopolamin-Konzentrationen von 0,07 $\pm$ 0,01 ng/kg erwies sich Scopolamin als wirksam, die durch Physostigmin verursachten unerwünschten Wirkungen, insbesondere Übelkeit und Erbrechen, zu unterdrücken. Statistisch signifikante Verhaltens- und Leistungsfähigkeitsänderungen ließen sich bei kombinierter Physostigmin-Scopolamin-Behandlung nicht nachweisen. Damit ist die erfindungsgemäß gestellte Aufgabe gelöst, die darin bestand, eine Arzneiform zu entwickeln, die mindestens ein Parasympathikomimetikum und mindestens ein Parasympathikolytikum enthält, ohne daß die für diese Arzneistoffe typischen Nebenwirkungen auftreten.

**Patentansprüche**

1. Transdermales Therapeutisches System zur Prophylaxe und zur Vorbehandlung einer Vergiftung durch hochtoxische phosphororganische Nervengifte mit einer Wirkstoffkombination aus mindestens einem Parasympathikomimetikum und mindestens einem Parasympathikolytikum, das zwei getrennte Reservoire für das Parasympathikomimetikum und das Parasympathikolytikum beinhaltet.

2. Transdermales Therapeutisches System nach Anspruch 1, **dadurch gekennzeichnet, dass** das Parasympathikolytikum aus der Gruppe der Tropanalkaloide, deren Salze und racemischen Gemische ausgewählt ist.

3. Transdermales Therapeutisches System nach Anspruch 1, **dadurch gekennzeichnet, dass** das Parasympathikomimetikum in einer Form vorliegt, bei welcher es indirekt wirksam ist.

4. Transdermales Therapeutisches System nach Anspruch 1 und 3, **dadurch gekennzeichnet, dass** das indirekt wirksame Parasympathikomimetikum Acetylcholinesterasehemmer umfasst.

5. Transdermales Therapeutisches System nach Anspruch 4, **dadurch gekennzeichnet, dass** der Acetylcholinesterasehemmer Physostigmin, Heptylphysostigmin, Neostigmin, Pyridostigmin, Galanthamin, Tetrahydroacridin und Velnacridin sowie deren Salze und racemische Gemische umfasst.

6. Transdermales Therapeutisches System nach einem oder mehreren der Ansprüche 1 bis 5, **dadurch gekennzeichnet, dass** das Parasympathikomimetikum Physostigmin und/oder dessen pharmazeutisch akzeptable Salze und das Parasympathikolytikum Scopolamin und/oder dessen pharmazeutischakzeptable Salze ist.

7. Transdermales Therapeutisches System nach einem oder mehreren der Ansprüche 1 bis 6, **dadurch gekennzeichnet, dass** die Reservoire eine so hohe Eigenklebrigkeit aufweisen, dass sie eine Haftkleberschicht darstellen.

8. Transdermales Therapeutisches System nach Anspruch 6 oder 7, **dadurch gekennzeichnet, dass** das Reservoir mit Scopolamin eine kleinere Fläche besitzt als das Reservoir mit Physostigmin und dass es einen wirkstofffreien Kleberand besitzt.

9. Transdermales Therapeutisches System, herstellbar durch die Arbeitsschritte:

   I. Herstellen eines wirkstofffreien Laminats durch Mischen von

   - 887,0 kg saurer Polyacrylatlösung (50%ig),
   - 10,1 kg basischem Methacrylat,
   - 50,4 kg Triacetin,
   - 0,508 kg Aluminiumacetylacetonat und
   - 37,8 kg Ethanol

   zu einer Lösung und Beschichten einer durch Silikonisierung wieder ablösbar gemachten Polyesterfolie mit der Lösung, so dass nach Abdampfen des Lösemittels das Kleberauftragsgewicht 120 g/cm$^2$ beträgt, Abdecken des Laminats mit in Längs- und Querrichtung elastischem Trägergewebe aus Polyester und Schneiden in Schmalrollen von 87 mm Breite,
   II. Herstellen eines scopolaminhaltigen Laminats durch Mischen von

   - 918,75 kg saurer Polyacrylatlösung (50%ig),
   - 84,38 kg 1-Dodecanol,
   - 3,68 kg Acetylaceton,
   - 3,38 kg Acetylacetonat und
   - 18,0 kg Scopolaminbase zu einer Lösung und Beschichten einer durch Silikonisierung wieder ablösbar gemachten Polyesterfolie mit der Lösung, so dass nach Verdampfen des Lösemittels das Kleberauftrags-

gewicht 150 g/cm$^2$ beträgt und Schneiden in Schmalrollen von 15 mm Breite,

III. Herstellen eines physostigminhaltigen Laminats durch Mischen von

- 542,2 kg saurer Polyacrylatlösung (50%ig),
- 125,8 kg 1-Dodecanol,
- 83,3 kg Physostigmin,
- 83,3 kg basischem Methacrylat,
- 16,6 kg Aluminiumacetylacetonat und
- 166,6 kg Ethanol

zu einer Lösung und Beschichten einer durch Silikonisierung wieder ablösbar gemachten Polyesterfolie mit dieser Lösung, so dass nach Verdampfen der Lösemittel das Kleberauftragsgewicht 240 g/cm$^2$ beträgt, Schneiden in Schmalrollen von 50 mm Breite,

IV. Entfernen der wieder ablösbaren silikonisierten Polyesterfolien von den scopolamin- und physostigminhaltigen Laminaten II und III und Transferieren von 15 x 50 mm$^2$ bzw. 50 x 50 mm$^2$ großen Rechtecken mit der klebenden Seite mittig und kantenparallel auf eine Polyesterfolie mit Silikonisierung von 87 mm Breite,

V. Entfernen der wieder ablösbar gemachten Polyesterfolie vom voirkstofffreien Laminat und kantengerades Kaschieren mit der klebenden Seite über die mit den Rechtecken versehene Bahn und

VI. Vereinzeln der Systeme durch ovales Ausstanzen.

10. Verwendung einer Wirkstoffkombination aus mindestens einem Parasympathikomimetikum und mindestens einem Parasympathikolytikum zur Herstellung eines transdermalen therapeutischen Systems, das zwei getrennte Reservoire für das Parasympathikomimetikum und das Parasympathikolytikum beinhaltet für die Prophylaxe und zur Vorbehandlung einer Vergiftung durch hochtoxische phosphororganische Cholinesterasehemmer.

11. Verwendung nach Anspruch 10, **dadurch gekennzeichnet, dass** es sich bei dem hochtoxischen phosphororganischen Cholinesterasehemmer um Nitrostigmin, Tabun, Sarin, Soman und/oder VX handelt.

12. Verwendung nach Anspruch 10 oder 11, **dadurch gekennzeichnet, dass** das Parasympathikolytikum aus der Gruppe der Tropanalkaloide, deren Salze und racemischen Gemische ausgewählt ist.

13. Verwendung nach einem oder mehreren der Ansprüche 10 bis 12, **dadurch gekennzeichnet, dass** das Parasympathikomimetikum in einer Form vorliegt, bei welcher es indirekt wirksam ist.

14. Verwendung nach einem oder mehreren der Ansprüche 10 bis 13, **dadurch gekennzeichnet, dass** das indirekt wirksame Parasympathikomimetikum Acetylcholinesterasehemmer umfasst.

15. Verwendung nach einem oder mehreren der Ansprüche 10 bis 14, **dadurch gekennzeichnet, dass** der Acetylcholinesterasehemmer Physostigmin, Heptylphysostigmin, Neostigmin, Pyridostigmin, Galanthamin, Tetrahydroacridin und Velnacridin sowie deren Salze und racemische Gemische umfasst.

16. Verwendung nach einem oder mehreren der Ansprüche 10 bis 15, **dadurch gekennzeichnet, dass** das Parasympathikomimetikum Physostigmin und/oder dessen pharmazeutisch akzeptable Salze und das Parasympathikolytikum Scopolamin und/oder dessen pharmazeutischakzeptable Salze sind.

17. Verwendung nach einem oder mehreren der Ansprüche 10 bis 16, **dadurch gekennzeichnet, dass** das Transdermale Therapeutische System eine kontinuierliche gleichmäßige Freisetzung der Wirkstoffe bis zu 72 Stunden aufweist.

18. Verfahren zur Herstellung eines Transdermalen Therapeutischen Systems mit einer Wirkstoffkombination aus Physostigmin und Scopolamin, das zwei getrennte Reservoire für Physostigmin und Scopolamin beinhaltet, durch die Arbeitsschritte:

I. Herstellen eines wirkstofffreien Laminats durch Mischen von

- 887,0 kg saurer Polyacrylatlösung (50%ig),
- 10,1 kg basischem Methacrylat,

- 50,4 kg Triacetin,
- 0,508 kg Aluminiumacetylacetonat und
- 37,8 kg Ethanol

zu einer Lösung und Beschichten einer durch Silikonisierung wieder ablösbar gemachten Polyesterfolie mit der Lösung, so dass nach Abdampfen des Lösemittels das Rleberauftragsgeovicht 120 g/cm$^2$ beträgt, Abdecken des Laminats mit in Längs- und Querrichtung elastischem Trägergewebe und Schneiden in Schmalrollen von 87 mm Breite,

II. Herstellen eines scopolaminhaltigen Laminats durch Mischen von

- 918,75 kg saurer Polyacrylatlösung (50%ig),
- 84,38 kg 1-Dodecanol,
- 3,68 kg Acetylaceton,
- 3,38 kg Acetylacetonat und
- 18,0 kg Scopolaminbase

zu einer Lösung und Beschichten einer durch Silikonisierung wieder ablösbar gemachten Polyesterfolie mit dieser Lösung, so dass nach dem Verdampfen des Lösemittels das Kleberauftragsgewicht 150 g/cm$^2$ beträgt und Schneiden in Schmalrollen von 15 mm Breite,

III. Herstellen eines physostigminhaltigen Laminats durch Mischen von

- 542,2 kg saurer Polyacrylatlösung (50%ig),
- 125,8 kg 1-Dodecanol,
- 83,3 kg Physostigmin,
- 83,3 kg basischem Methacrylat,
- 16,6 kg Aluminiumacetylacetonat und
- 166,6 kg Ethanol

zu einer Lösung und Beschichten einer durch Silikonisierung wieder ablösbar gemachten Polyesterfolie mit dieser Lösung, so dass nach Verdampfen der Lösemittel das Kleberauftragsgewicht 240 g/cm$^2$ beträgt und Schneiden in Schmalrollen von 50 mm Breite,

IV. Entfernen der wieder ablösbaren silikonisierten Polyesterfolien von den scopolamin- und physostigminhaltigen Laminaten II und III und Transferieren von 15 x 50 mm$^2$ bzw. 50 x 50 mm$^2$ großen Rechtecken mit der klebenden Seite mittig und kantenparallel auf eine Polyesterfolie mit Silikonisieung von 87 mm Breite,

V. Entfernen der wieder ablösbare gemachten Polyesterfolie vom wirkstofffreien Laminat und kantengerades Kaschieren mit der klebenden Seite über die mit den Rechtecken versehene Bahn und

VI. Vereinzeln der Systeme durch Ausstanzen.

## Claims

1. Transdermal therapeutic system for the prophylaxis and pretreatment of a poisoning caused by highly toxic organophosphorous neurotoxins, comprising an active substance combination of at least one parasympathomimetic and at least one parasympatholytic, which transdermal therapeutic system has two separate reservoirs for said parasympathomimetic and said parasympatholytic.

2. Transdermal therapeutic system according to claim 1, **characterised in that** the parasympatholytic is selected from the group of the tropane alkaloids, their salts and racemic mixtures thereof.

3. Transdermal therapeutic system according to claim 1, **characterised in that** the parasympathomimetic is present in a form in which it is indirectly effective.

4. Transdermal therapeutic system according to claims 1 and 3, **characterised in that** the indirectly effective parasympathomimetic comprises acetylcholinesterase inhibitors.

5. Transdermal therapeutic system according to claim 4, **characterised in that** the acetylcholinesterase inhibitor comprises physostigmine, heptylphysostigmine, neostigmine, pyridostigmine, galanthamine, tetrahydroacridine and velnacridine, as well as the salts and racemic mixtures thereof.

**6.** Transdermal therapeutic system according to one or more of claims 1 to 5, **characterised in that** the parasympathomimetic is physostigmine and/or the pharmaceutically acceptable salts thereof, and that the parasympatholytic is scopolamine and/or the pharmaceutically acceptable salts thereof.

**7.** Transdermal therapeutic system according to one or more of claims 1 to 6, **characterised in that** the reservoirs have an inherent tack that is so high that the reservoirs constitute a pressure-sensitive adhesive layer.

**8.** Transdermal therapeutic system according to claim 6 or 7, **characterised in that** the reservoir which contains scopolamine has a smaller surface area than the reservoir containing physostigmine, and that it has an active substance-free adhesive margin.

**9.** Transdermal therapeutic system which can be produced by means of the production steps of:

I. preparing an active substance-free laminate by mixing

- 887.0 kg acidic polyacrylate solution (50%)
- 10.1 kg basic methacrylate
- 50.4 kg triacetin,
- 0.508 kg aluminium acetylacetonate and
- 37.8 kg ethanol

to form a solution, and coating a polyester film, which has been rendered detachable by means of siliconisation, with this solution such that, after evaporation of the solvent, the adhesive coating weight is 120 g/cm$^2$; covering the laminate with a supporting fabric of polyester which is elastic in the longitudinal and transverse direction; and cutting into narrow rolls having a width of 87 mm;
II. preparing a scopolamine-containing laminate by mixing

- 918.75 kg acidic polyacrylate solution (50%)
- 84.38 kg 1-dodecanol
- 3.68 kg acetylacetone
- 3.38 kg acetylacetonate and
- 18.0 kg scopolamine base

to form a solution, and coating a polyester film, which has been rendered detachable by means of siliconisation, with this solution such that, after evaporation of the solvent, the adhesive coating weight is 150 g/cm$^2$; and cutting into narrow rolls having a width of 15 mm;
III. preparing a physostigmine-containing laminate by mixing

- 542.2 kg acidic polyacrylate solution (50%)
- 125.8 kg 1-dodecanol
- 83.3 kg physostigmine
- 83.3 kg basic methacrylate
- 16.6 kg aluminium acetylacetonate and
- 166.6 kg ethanol

to form a solution, and coating a polyester film, which has been rendered detachable by means of siliconisation, with this solution such that, after evaporation of the solvents, the adhesive coating weight is 240 g/cm$^2$; cutting into narrow rolls having a width of 50 mm;

IV. removing the detachable siliconised polyester films from the scopolamine-containing and physostigmine-containing laminates II and III, and transferring the adhesive side of rectangles having a size of 15 x 50 mm$^2$ and 50 x 50 mm$^2$, respectively, onto a polyester film, centrally and in parallel to the edges thereof, said polyester film having been siliconised and having a width of 87 mm;
V. removing the polyester film, which has been rendered detachable, from the active substance-free laminate, and laminating the adhesive side, along the edges, onto the web provided with the rectangles; and
VI. separating the systems by punching out ovals.

**10.** Use of an active substance combination of at least one parasympathomimetic and at least one parasympatholytic for producing a transdermal therapeutic system that contains two separate reservoirs for said parasympathomimetic and said parasympatholytic, for the prophylaxis and pretreatment of a poisoning caused by highly toxic organophosphorous cholinesterase inhibitors.

11. Use according to claim 10, **characterised in that** the highly toxic organophosphorous cholinesterase inhibitor is nitrostigmine, tabun, sarin, soman and/or VX.

12. Use according to claim 10 or 11, **characterised in that** the parasympatholytic is selected from the group of the tropane alkaloids, their salts and racemic mixtures thereof.

13. Use according to one or more of claims 10 to 12, **characterised in that** the parasympathomimetic is present in a form in which it is indirectly effective.

14. Use according to one or more of claims 10 to 13, **characterised in that** the indirectly effective parasympathomimetic comprises acetylcholinesterase inhibitors.

15. Use according to one or more of claims 10 to 14, **characterised in that** the acetylcholinesterase inhibitor comprises physostigmine, heptylphysostigmine, neostigmine, pyridostigmine, galanthamine, tetrahydroacridine and velnacridine, as well as the salts and racemic mixtures thereof.

16. Use according to one or more of claims 10 to 15, **characterised in that** the parasympathomimetic is physostigmine and/or the pharmaceutically acceptable salts thereof, and that the parasympatholytic is scopolamine and/or the pharmaceutically acceptable salts thereof.

17. Use according to one or more of claims 10 to 16, **characterised in that** the transdermal therapeutic system has a continuous and uniform release of the active substances for a period of up to 72 hours.

18. Method of producing a transdermal therapeutic system comprising an active substance combination of physostigmine and scopolamine and containing two separate reservoirs for physostigmine and scopolamine, by means of the production steps of

    I. preparing an active substance-free laminate by mixing

        - 887.0 kg acidic polyacrylate solution (50%)
        - 10.1 kg basic methacrylate
        - 50.4 kg triacetin,
        - 0.508 kg aluminium acetylacetonate and
        - 37.8 kg ethanol
        to form a solution, and coating a polyester film, which has been rendered detachable by means of siliconisation, with this solution such that, after evaporation of the solvent, the adhesive coating weight is 120 g/cm$^2$; covering the laminate with a supporting fabric which is elastic in the longitudinal and transverse direction; and cutting into narrow rolls having a width of 87 mm;

    II. preparing a scopolamine-containing laminate by mixing

        - 918.75 kg acidic polyacrylate solution (50%)
        - 84.38 kg 1-dodecanol
        - 3.68 kg acetylacetone
        - 3.38 kg acetylacetonate and
        - 18.0 kg scopolamine base
        to form a solution, and coating a polyester film, which has been rendered detachable by means of siliconisation, with this solution such that, after evaporation of the solvent, the adhesive coating weight is 150 g/cm$^2$; and cutting into narrow rolls having a width of 15 mm;

    III. preparing a physostigmine-containing laminate by mixing

        - 542.2 kg acidic polyacrylate solution (50%)
        - 125.8 kg 1-dodecanol
        - 83.3 kg physostigmine

- 83.3 kg basic methacrylate
- 16.6 kg aluminium acetylacetonate and
- 166.6 kg ethanol

to form a solution, and coating a polyester film, which has been rendered detachable by means of siliconisation, with this solution such that, after evaporation of the solvents, the adhesive coating weight is 240 g/cm$^2$; and cutting into narrow rolls having a width of 50 mm;

IV. removing the detachable siliconised polyester films from the scopolamine-containing and physostigmine-containing laminates II and III, and transferring the adhesive side of rectangles having a size of 15 x 50 mm$^2$ and 50 x 50 mm$^2$, respectively, onto a polyester film, centrally and in parallel to the edges thereof, said polyester film having been siliconised and having a width of 87 mm;

V. removing the polyester film, which has been rendered detachable, from the active substance-free laminate, and laminating the adhesive side, along the edges, onto the web provided with the rectangles; and

VI. separating the systems by punching.

## Revendications

1. Système thérapeutique transdermique destiné à la prophylaxie et au prétraitement d'un empoisonnement par des poisons nerveux organophosphorés hautement toxiques avec une combinaison de substances actives d'au moins un parasympathicomimétique et d'au moins un parasympathicolytique qui comporte deux réservoirs séparés pour le parasympathicomimétique et le parasympathicolytique.

2. Système thérapeutique transdermique selon la revendication 1, **caractérisé en ce que** le parasympathicolytique est choisi dans le groupe des alcaloïdes tropaniques, leurs sels et leurs mélanges racémiques.

3. Système thérapeutique transdermique selon la revendication 1, **caractérisé en ce que** le parasympathicomimétique se trouve sous une forme dans laquelle il est indirectement actif.

4. Système thérapeutique transdermique selon la revendication 1 et 3, **caractérisé en ce que** le parasympathicomimétique indirectement actif est un inhibiteur de l'acétylcholinestérase.

5. Système thérapeutique transdermique selon la revendication 4, **caractérisé en ce que** l'inhibiteur de l'acétylcholinestérase comprend la physostigmine, l'heptylphysostigmine, la néostigmine, la pyridostigmine, la galanthamine, la tétrahydroacridine et la velnacridine ainsi que leurs sels et mélanges racémiques.

6. Système thérapeutique transdermique selon l'une ou plusieurs des revendications 1 à 5, **caractérisé en ce que** le parasympathicomimétique est la physostigmine et/ou ses sels pharmaceutiquement acceptables et le parasympathicolytique est la scopolamine et/ou ses sels pharmaceutiquement acceptables.

7. Système thérapeutique transdermique selon l'une ou plusieurs des revendications 1 à 6, **caractérisé en ce que** les réservoirs présentent une adhésivité propre tellement élevée qu'ils représentent une couche autoadhésive.

8. Système thérapeutique transdermique selon la revendication 6 ou 7, **caractérisé en ce que** le réservoir avec la scopolamine présente une surface plus petite que le réservoir avec la physostigmine et **en ce qu'**il présente un bord adhésif sans substance active.

9. Système thérapeutique transdermique, pouvant être préparé par les étapes de travail :

I. préparation d'un laminé exempt de substance active par mélange de

- 887,0 kg d'une solution acide de polyacrylate (à 50%),
- 10,1 kg de méthacrylate basique,
- 50,4 kg de triacétine,
- 0,508 kg d'acétylacétonate d'aluminium et
- 37,8 kg d'éthanol

en une solution et revêtement d'une feuille de polyester rendue réamovible par un revêtement siliconé par la solution, de manière telle qu'après l'évaporation du solvant, le poids appliqué d'adhésif est de 120 g/cm$^2$,

recouvrement du laminé avec un tissu support en polyester élastique dans le sens longitudinal et transversal et découpe en rouleaux étroits d'une largeur de 87 mm,

II. préparation d'un laminé contenant de la scopolamine par mélange de

- 918,75 kg d'une solution acide de polyacrylate (à 50%),
- 84,38 kg de 1-dodécanol,
- 3,68 kg d'acétylacétone,
- 3,38 kg d'acétylacétonate et
- 18,0 kg de scopolamine-base

en une solution et revêtement d'une feuille de polyester rendue réamovible par un revêtement siliconé par la solution, de manière telle qu'après l'évaporation du solvant, le poids appliqué d'adhésif est de 150 g/cm$^2$ et découpe en rouleaux étroits d'une largeur de 15 mm,

III. préparation d'un laminé contenant de la physostigmine par mélange de

- 542,2 kg d'une solution acide de polyacrylate (à 50%),
- 125,8 kg de 1-dodécanol,
- 83,3 kg de physostigmine,
- 83,3 kg de méthacrylate basique,
- 16,6 kg d'acétylacétonate d'aluminium et
- 166,6 kg d'éthanol

en une solution et revêtement d'une feuille de polyester rendue réamovible par un revêtement siliconé par cette solution, de manière telle qu'après l'évaporation des solvants, le poids appliqué d'adhésif est de 240 g/cm$^2$ et découpe en rouleaux étroits d'une largeur de 50 mm,

IV. élimination des feuilles de polyester siliconées rendues réamovibles des laminés II et III contenant de la scopolamine et de la physostigmine et transfert de rectangles d'une taille de 15 x 50 mm$^2$ respectivement de 50 x 50 mm$^2$ avec le côté adhésif, au centre et le longs des bords sur une feuille de polyester avec un revêtement siliconé d'une largeur de 87 mm,

V. élimination de la feuille de polyester rendue réamovible du laminé exempt de substances actives et contrecollage le long des bords avec le côté adhésif sur la bande pourvue des rectangles et

VI. individualisation des systèmes par découpe en ovale.

10. Utilisation d'une combinaison de substances actives d'au moins un parasympathicomimétique et d'au moins un parasympathicolytique pour la préparation d'un système thérapeutique transdermique, qui comporte deux réservoirs séparés pour le parasympathicomimétique et le parasympathicolytique destiné à la prophylaxie et au prétraitement d'un empoisonnement par un inhibiteur de cholinestérase organophosphoré hautement toxique.

11. Utilisation selon la revendication 10, **caractérisée en ce qu'**il s'agit, pour l'inhibiteur de cholinestérase organophosphoré hautement toxique, de nitrostigmine, de tabun, de sarin, de soman et/ou de VX.

12. Utilisation selon la revendication 10 ou 11, **caractérisée en ce que** le parasympathicolytique est choisi dans le groupe des alcaloïdes tropaniques, leurs sels et leurs mélanges racémiques.

13. Utilisation selon l'une ou plusieurs des revendications 10 à 12, **caractérisée en ce que** le parasympathicomimétique se trouve sous une forme dans laquelle il est indirectement actif.

14. Utilisation selon l'une ou plusieurs des revendications 10 à 13, **caractérisée en ce que** le parasympathicomimétique indirectement actif est un inhibiteur de l'acétylcholinestérase.

15. Utilisation selon l'une ou plusieurs des revendications 10 à 14, **caractérisée en ce que** l'inhibiteur de l'acétylcholinestérase comprend la physostigmine, l'heptylphysostigmine, la néostigmine, la pyridostigmine, la galanthamine, la tétrahydroacridine et la velnacridine ainsi que leurs sels et mélanges racémiques.

16. Utilisation selon l'une ou plusieurs des revendications 10 à 15, **caractérisée en ce que** le parasympathicomimétique est la physostigmine et/ou ses sels pharmaceutiquement acceptables et le parasympathicolytique est la scopolamine et/ou ses sels pharmaceutiquement acceptables.

**17.** Utilisation selon l'une ou plusieurs des revendications 10 à 16, **caractérisée en ce que** le système thérapeutique transdermique présente une libération régulière continue des substances actives pendant jusqu'à 72 heures.

**18.** Procédé pour la préparation d'un système thérapeutique transdermique présentant une combinaison de substances actives de physostigmine et de scopolamine, qui comporte deux réservoirs séparés pour la physostigmine et de scopolamine, par les étapes de travail :

    I. préparation d'un laminé exempt de substance active par mélange de

        - 887,0 kg d'une solution acide de polyacrylate (à 50%),
        - 10,1 kg de méthacrylate basique,
        - 50,4 kg de triacétine,
        - 0,508 kg d'acétylacétonate d'aluminium et
        - 37,8 kg d'éthanol

    en une solution et revêtement d'une feuille de polyester rendue réamovible par un revêtement siliconé par la solution, de manière telle qu'après l'évaporation du solvant, le poids appliqué d'adhésif est de 120 g/cm$^2$, recouvrement du laminé avec un tissu support élastique dans le sens longitudinal et transversal et découpe en rouleaux étroits d'une largeur de 87 mm,

    II. préparation d'un laminé contenant de la scopolamine par mélange de

        - 918,75 kg d'une solution acide de polyacrylate (à 50%),
        - 84,38 kg de 1-dodécanol,
        - 3,68 kg d'acétylacétone,
        - 3,38 kg d'acétylacétonate et
        - 18,0 kg de scopolamine-base

    en une solution et revêtement d'une feuille de polyester rendue réamovible par un revêtement siliconé par cette solution, de manière telle qu'après l'évaporation du solvant, le poids appliqué d'adhésif est de 150 g/cm$^2$ et découpe en rouleaux étroits d'une largeur de 15 mm,

    III. préparation d'un laminé contenant de la physostigmine par mélange de

        - 542,2 kg d'une solution acide de polyacrylate (à 50%),
        - 125,8 kg de 1-dodécanol,
        - 83,3 kg de physostigmine,
        - 83,3 kg de méthacrylate basique,
        - 16,6 kg d'acétylacétonate d'aluminium et
        - 166,6 kg d'éthanol

    en une solution et revêtement d'une feuille de polyester rendue réamovible par un revêtement siliconé par cette solution, de manière telle qu'après l'évaporation des solvants, le poids appliqué d'adhésif est de 240 g/cm$^2$ et découpe en rouleaux étroits d'une largeur de 50 mm,

    IV. élimination des feuilles de polyester siliconées rendues réamovibles des laminés II et III contenant de la scopolamine et de la physostigmine et transfert de rectangles d'une taille de 15 x 50 mm$^2$ respectivement de 50 x 50 mm$^2$ avec le côté adhésif, au centre et le long des bords sur une feuille de polyester avec un revêtement siliconé d'une largeur de 87 mm,
    V. élimination de la feuille de polyester rendue réamovible du laminé exempt de substances actives et contrecollage le long des bords avec le côté adhésif sur la bande pourvue des rectangles et
    VI. individualisation des systèmes par découpe.

FIG. 1

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE OS4115558 A

- DE 3843239

**In der Beschreibung aufgeführte Nicht-Patentliteratur**

- **Leadbeater, L.** *Chem. in Brit.,* 1988, vol. 24, 683
- **Fleischer, J.H. ; Harris, L.W.** *Biochem. Pharmacol.,* 1965, vol. 14, 641
- **Berry, W.K. ; Davies, D.R.** *Biochem, Pharmacol,* 1970, vol. 19, 927

- **Levy et al.** *Proc. 4th Int. Symp. Protection Against Chemical Warfare Agents,* 1992, 277-284
- **von Levy et al.** A Long-Acting Transdermal System for the Treatment of Organophosphate Poisoning. *Proc. 3rd Int. Symp. Protection Against Chemical Warfare Agents,* 1989, 151-156